# EUROPÄISCHE PATENTANMELDUNG

(11) **EP 1 731 503 A2**
(43) Veröffentlichungstag der Anmeldung: **13.12.2006**
(21) Anmeldenummer: 06011032.7
(22) Anmeldetag: 30.05.2006
(51) Int. Cl.: C07C 253/14, C07C 255/40

(54) **Verfahren zur Herstellung von substituierten Benzoylcyaniden**

(30) Priorität: 11.06.2005 DE 102005027149
(71) Anmelder: Saltigo GmbH, 51369 Leverkusen (DE)
(72) Erfinder: Job, Andreas, Dr., 50858 Köln (DE); Schlummer, Björn, Dr., 53115 Bonn (DE)
(74) Vertreter: Pettrich, Klaus-Günter

(57) **Zusammenfassung**

Verfahren zur Herstellung von substituierten Benzoylcyaniden der allgemeinen Formel (I) 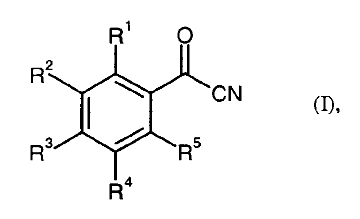 wobei R¹, R², R³, R⁴, R⁵ unabhängig voneinander für Wasserstoff, Chlor, Brom, Jod, Fluor, einen C₁-C₈-Alkyl-, Aryl-, Arylalkyl-, C₁-C₈-Alkoxy-oder C₁-C₈-Alkylmercapto-Rest oder für -CN, -COOR⁶, -CONR₂⁷ ,-SO₃R⁸, oder -SO₂NR₂⁹ stehen, wobei R⁶, R⁷, R⁸, R⁹ unabhängig voneinander einem C₁-C₈-Alkylrest entsprechen,
durch
a) Umsetzung von Benzoylchloriden der allgemeinen Formel (II) 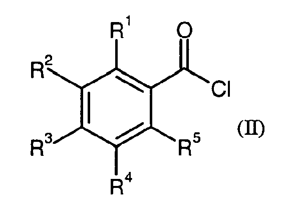 in der
R¹, R², R³, R⁴, R⁵
die gleiche Bedeutung wie in Formel I haben,

mit 0,9-1,4 Moläquivalenten Kupfercyanid ohne weiteres Lösungsnüttel, gegebenenfalls unter erhöhten Druck, unter Inertgasatmosphäre bei einer Reaktionstemperatur zwischen 150 und 165°C,
b) wobei nach einer Reaktionszeit von maximal 5 Stunden dem Reaktionsgemisch nach Abkühlung auf eine Temperatur unter 100°C ein aprotisches organisches Lösungsmittel zur Ausfällung des entstandenen Kupfersalzes zugefügt wird und
c) nach Abfiltration des ausfällten Kupfersalzes das verbliebene Filtrat auf eine Temperatur zwischen -40 und +20°C zur Auskristallisation des Rohproduktes abgekühlt wird und
d) das auskristallisierte Rohprodukt vom aprotischen organischen Lösungsmittel abgetrennt und gegebenenfalls getrocknet wird.

## Beschreibung

Die vorliegende Erfindung betrifft ein verbessertes Verfahren zur Herstellung von substituierten, bevorzugt halogensubstituierten Benzoylcyaniden.

Eine Übersicht über die Herstellung von Benzoylcyaniden wird in Angew. Chem., Int. Ed. Engl. 1982, 21, 36, Schema 1 gegeben. Darin werden ausgehend von Benzoylchloriden zwei generelle Wege beschrieben. Zunächst können Benzoylcyanide aus den entsprechenden Chloriden erhalten werden, indem sie zum Beispiel nach Synthesis 1979, 204 mit Trimethylsilylcyanid umgesetzt werden. Darüber hinaus wird die Umsetzung von Benzoylchloriden mit Schwermetallcyaniden beschrieben, die allerdings in dem Bezug als nachteilig beschrieben wird, da man die Reaktionsmischung über Stunden auf Temperaturen von 120-160°C erhitzen muss, was die Begünstigung der Bildung von Nebenkomponenten vermuten lässt. Als vorteilhafter dagegen wird darin ein Verfahren beschrieben, bei dem Benzoylchloride mit Kupfercyanid unter Zusatz von Acetonitril schon bei 80°C mit einer Ausbeute von bis zu 75% umgesetzt werden können. Dem Fachmann erscheint hier allerdings die Abtrennung des als Reagenz eingesetzten Kupfercyanids, sowie bei der Reaktion entstehendem Kupferchlorids mittels einfacher Filtration als schwierig, da diese Salze mit eben dem eingesetzten Acetonitril bekanntermaßen lösliche Komplexe bilden. Mit einem eher exotischen und technisch nicht verfügbaren Cyanid, dem Thalliumcyanid, werden nach vorgenanntem Artikel sogar schon bei 20°C Ausbeuten an Benzoylcyaniden von 89% erreicht.

Aus DE-A 3 035 021, DE-A 4 311 722 und EP-A 1 316 613 sind Reaktion von Benzoylchloriden mit Kupfercyanid unter Zusatz von Acetonitril bekannt, bei denen die erhaltenen Rohprodukte nach Trennung von den Kupfersalzen durch Filtration abgetrennt werden und anschließend gegebenenfalls noch nach Wäschen mit Wasser zur Reinigung destilliert werden. Diese Vorgehensweise wäre für eine technische Umsetzung höchst unwirtschaftlich und zudem nur anwendbar für Substanzen, die sich auch unter technischen Bedingungen destillieren lassen.

In DE-A 2 624 891, DE-A 2 708 182 und US-A 4 209 462 sind Herstellungsverfahren von Benzoylcyaniden aus Benzoylchloriden unter Verwendung von überstöchiometrischen Mengen Natriumcyanid und unterstöchiometischen Mengen von Kupfer, Kupferoxid oder Kupfercyanid und gegebenenfalls noch Zusätzen von Acetonitril und gegebenenfalls noch weiteren Lösungsmitteln offengelegt. Allen diesen Vorgehensweisen ist gemeinsam, dass die erhaltenen Rohprodukte in einem separaten Schritt zur Reinigung destilliert werden müssen. Nachteilig an dieser Vorgehensweise ist die Beschränkung auf destillierbare Benzoylcyanide, sowie bei DE-A 2 624 891 und DE-A 2 708 182 die eingeschränkte Recyclierbarkeit der Abfallströme, da diese in den aufgeführten Beispielen aus Lösungsmittelgemischen bestehen.

Aus US - 2003158435 ist ein Verfahren zur Herstellung von Benzoylcyanid aus Benzyolchlorid in einem wässrigen Zweiphasensystem bekannt. Dabei werden Natriumcyanid, Natriumiodid und Natronlauge als Reagenzien eingesetzt. Diese Vorgehensweise ist insofern von Nachteil, da sie sich nur zur Herstellung von wasserstabilen Verbindungen nutzen lässt.

In J. Fluorine Chem. 1993, 61, 117 ist die Reaktion von Benzoylchloriden mit 1,5 Äquivalenten Kupfercyanid und katalytischen Mengen Phosphorpentaoxid in Toluol beschrieben, wobei die Reaktionsmischung über 40 Stunden auf Rückflusstemperatur erhitzt wird. Dieses Vorgehen ist sowohl durch den Einsatz von einem Überschuss von 0,5 Äquivalenten Cyanidreagents, den Zusatz von reaktivem Phosphorpentoxid, als auch wegen der hohen Temperaturbelastung von Nachteil, da sich in diesem Fall eine Vielzahl von Nebenreaktionen vermuten lässt. Dieses wird dadurch bestätigt, dass die erhaltenen Rohprodukte sehr aufwendig durch eine Filtration, anschließendes Waschen mit Wasser, eine chromatographische Reinigung und noch eine Umkristallisation gereinigt werden müssen. Die erhaltenen Ausbeuten für die beschriebenen Beispiele (5 Stück) liegen erwartungsgemäß niedrig bei 45-76 %.

In GB-A 2 395 483 ist die Herstellung von 2,3-Dichlorbenzoylcyanid derart beschrieben, dass 2,3-Dichlorbenzoylchlorid mit 1,4 Äquivalenten Kupfercyanid bei 160-165°C 6 Stunden lang umgesetzt wird. Die Aufarbeitung beinhaltet zunächst das Verdünnen mit Toluol, eine Filtration und nach Abdestillieren des Toluols eine Umkristallisation des Rohprodukts. Nachteilig an diesem Herstellungsverfahren ist die lange Temperaturbelastung sowohl durch die Reaktion, als auch durch das Abdestillieren des zugegebenen Toluols, welches im technischen Maßstab ebenfalls einiger Stunden bedarf. Die Reinheit des Produkts wird nach Gaschromatographischer Analyse mit 97% angegeben, wobei sich die üblichen Nebenkomponenten in dieser Reaktion wie das entsprechende Anhydrid oder die Säure üblicherweise nicht gut mittels Gaschromatographie detektieren lassen. Das Nachstellen dieser Reaktion hat zu deutlichem Nebenkomponentenanteil im Produkt geführt.

Es bestand daher die Aufgabe ein Verfahren zur wirtschaftlichen Herstellung von substituierten, insbesondere halogensubstituierten Benzoylcyaniden in einer guten Reinheit zu entwickeln.

Es wurde nun ein Verfahren zur Herstellung von substituierten Benzoylcyaniden der allgemeinen Formel I 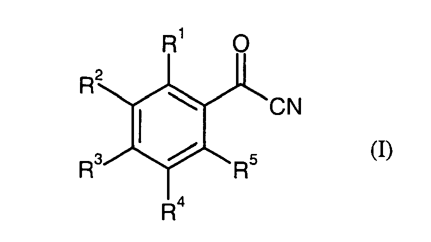 wobei R¹, R², R³, R⁴, R⁵ unabhängig voneinander für Wasserstoff, Chlor, Brom, Jod, Fluor, einen C1-C8-Alkyl-, Aryl-, Arylalkyl-, C1-C8-Alkoxy-oder C1-C8-Alkylmercapto-Rest oder für -CN, - COOR⁶, -CONR₂⁷,- SO ₃R⁸, oder -SO ₂NR₂⁹ stehen, wobei R⁶, R⁷, R⁸, R⁹ unabhängig voneinander einem C 1- C8-Alkylrest entsprechen,
durch Umsetzung von Benzoylchloriden der allgemeinern Formel II 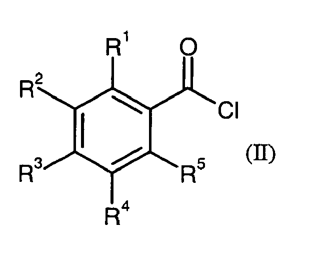 in der
- R¹, R², R³, R⁴, R⁵: die gleiche Bedeutung wie in Formel I haben,
mit Kupfercyanid, gefunden, bei dem die Reaktion in Substanz, d.h. unter Abwesenheit von organischen Lösungsmitteln, sowie sonstigen Zusatzstoffen, verläuft, wobei zur Aufarbeitung höchstens ein inertes organisches Lösungsmittel bzw. ein Lösungsmittelgemisch (ggf. "Destillationsschnitt" in üblicher technischer Qualität) eingesetzt wird und die Isolierung des Produkts nach Abtrennung der anorganischen Nebenprodukte durch Filtration durch Kristallisation aus dem Lösungsmittel erfolgt.

Das erhaltene Produkt weist eine Reinheit von mindestens 96 % bezogen auf alle Inhaltsstoffe auf.

Gegenstand der Erfindung ist daher ein Verfahren zur Herstellung von halogensubstituierten Benzoylcyaniden der allgemeinen Formel (I) 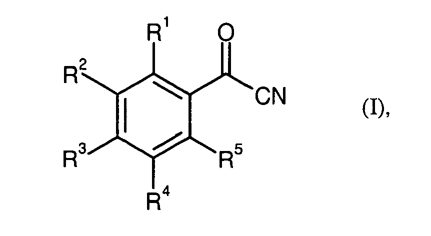 wobei R¹, R², R³, R⁴, R⁵ unabhängig voneinander für Wasserstoff, Chlor, Brom, Jod, Fluor, einen C₁-C₈-Alkyl-, Aryl-, Arylalkyl-, C₁-C₈-Alkoxy-oder C₁-C₈-Alkylmercapto-Rest oder für -CN, -COOR⁶, -CONR₂⁷ ,-SO₃R⁸, oder -SO ₂NR₂⁹ stehen, wobei R⁶, R⁷, R⁸, R⁹ unabhängig voneinander für einen C₁-C₈-Alkylrest stehen,
stehen,
durch
a) Umsetzung von Benzoylchloriden der allgemeinen Formel (II) 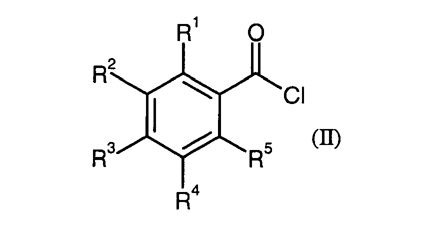 in der
   - R¹, R², R³, R⁴, R⁵: die gleiche Bedeutung wie in Formel I haben,
   mit 0,9-1,4 Moläquivalenten Kupfercyanid ohne weiteres Lösungsmittel, gegebenenfalls unter erhöhtem Druck, unter Inertgasatmosphäre bei einer Reaktionstemperatur zwischen 150 und 165°C,
b) wobei nach einer Reaktionszeit von maximal 5 Stunden dem Reaktionsgemisch nach Abkühlung auf eine Temperatur unter 100°C ein aprotisches organisches Lösungsmittel zur Ausfällung des entstandenen Kupfersalzes zugefügt wird und
c) nach Abfiltration des ausfällten Kupfersalzes das verbliebene Filtrat auf eine Temperatur zwischen -40 und +20°C zur Auskristallisation des Rohproduktes abgekühlt wird und
d) das auskristallisierte Rohprodukt vom aprotischen organischen Lösungsmittel abgetrennt und gegebenenfalls getrocknet wird.

Beim erfindungsgemäßen Verfahren wird das substituierte Benzoylchlorid mit Kupfercyanid in Substanz für maximal 5 Stunden, bevorzugt für maximal 4 Stunden bei einer Temperatur im Bereich von 150 bis165°C, bevorzugt 155-160°C zur Reaktion gebracht. Vergleichsexperimente, insbesondere nach GB-A- 2395483 haben gezeigt, dass mit zunehmender Reaktionszeit die Bildung von Nebenkomponenten stark zunimmt, was sich negativ auf die erhaltene Produktqualität auswirkt. Überraschenderweise kann man erfindungsgemäß unter Einhaltung der Reaktionszeiten eine deutlich geringere Menge an Cyanierungsreagens von ca. 1,1 Äquivalenten anstatt wie in GB 2395483 beschrieben 1,4 Äquivalente bzw. in J. Fluorine Chem. 1993, 61, 117 beschrieben 1,5 Äquivalente verwenden, ohne Einbussen in der Produktausbeute zu beobachten. Da es sich bei den Reagenzien um sehr giftige Substanzen handelt, erfolgt durch diese erfindungsgemäße Vorgehensweise eine deutlich geringere Umweltbelastung durch einen deutlich verringerten Entsorgungsaufwand.

Üblicherweise werden 0,9 bis 1,4 Moläquivalente (bezogen auf das Benzoylchlorid), bevorzugt 1,0 bis 1,3, besonders bevorzugt 1,0- 1,1 Moläquivalente Kupfercyanid eingesetzt.

Da Edukte und Produkte wasserempfindlich sind, wird unter Inertgasatmosphäre gearbeitet. Als Inertgas werden dabei Stickstoff oder Edelgase wie Helium oder Argon eingesetzt.

Als substituierte Benzoylchloride können erfindungsgemäß solche hergestellt werden, bei denen unabhängig voneinander R1, R2 ,R3 ,R4 und R5 die in Formel I beschriebene Bedeutung haben. Als C1-C8-Alkylreste sind dabei Methyl-, Ethyl-, Propyl- und Butylreste, als Arylreste Phenyl-oder substituierte Phenylreste, als Arylalkylreste Benzyl- oder substituierte Benzylreste bevorzugt.

Besonders eignet sich das erfindungsgemäße Verfahren zu Herstellung von halogensubstituierten Benzoylcyaniden , d.h., dass hierbei mindestens einer der Reste R1, R2, R3, R4 oder R5 für Chlor, Brom, Jod oder Fluor steht. Bevorzugte Verbindungen sind dabei die 2,3- 2,4-, 2,5-, oder 2,6-, oder 3,4-Dihalogenbenzoylcyanide, insbesondere 2,3-Dichlorbenzoylcyanid, 2,4-Dichlorbenzoylcyanid, 2,5-Dichlorbenzoylcyanid, 2,6-Dichlorbenzoylcyanid, 3,4-Dichlorbenzoylcyanid sowie 4-Brom-2-chlorbenzoylcyanid und 4-Brom-2-fluorbenzoylcyanid, die 2-, 3- und 4-Monohalogenbenzoylcyanide, insbesondere 4-Brombenzoylcyanid und 4-Fluorbenzoylcyanid, die 2,3,4-, 2,3,5-, 2,4,6-, 2,3,6-Trihalogenbenzoylcyanide, insbesondere 2,3,4-Trichlorbenzoylcyanid und 4-Brom-2,6-dichlorbenzoylcyanid.

Überraschend kann zur Abtrennung der anorganischen Kupfersalze die Reaktionsmischung direkt mit dem zur Kristallisation geeigneten Lösungsmittel bzw. Lösungsmittelgemisch verdünnt werden und nach Filtration von den Kupfersalzen, das Produkt aus dieser Lösung kristallisiert werden. Durch diese Vorgehensweise entfallen weitere Schritte, die durch eine längere Verweilzeit bzw. gleichzeitige weitere Temperaturbelastung des Rohprodukts wie zum Beispiel in GB-A- 2395483 beschrieben, beim Abdestillieren des zur Abtrennung des entstandenen Kupfersalzes verwendeten Toluols, oder in J. Fluorine Chem. 1993, 61, 117 beschrieben, wo zusätzlich zur Toluoldestillation zuvor noch Wasserwäschen durchgeführt werden, die Bildung von Nebenkomponenten begünstigen und daher die Produktqualität deutlich negativ beeinflussen. Neben der verbesserten Produktqualität wird zusätzlich noch die Einsparung mindestens eines Lösungsmittels, welches üblicherweise in deutlich größeren Mengen, als das Produkt selbst verwendet wird, erzielt. Die damit verbundene Einsparung an Rohmaterialien, sowie deren Beseitigung stellen einen wirksamen Effekt zur Verbesserung der Umweltfreundlichkeit des Verfahrens, sowie eine damit verbundene entscheidend verbesserte Wirtschaftlichkeit dar.

Das zur Abtrennung von den Kupfersalzen, sowie zur Kristallisation verwendete inerte aprotische organische Lösungsmittel gehört üblicherweise zur Gruppe der unpolaren aliphatischen C₂-C₈₋Alkane, wie z. B. n-Hexan und dessen Isomeren, n-Heptan und dessen Isomeren, n-Octan und dessen Isomeren, sowie Cycloalkane wie Cyclopentan, Cyclohexan, Cycloheptan, Cyclooctan und auch mit Alkylgruppen wie Methyl, Ethyl, Propyl, Isopropyl, n-Butyl, sec-Butyl, tert.-Butyl substituierte Cycloalkane. Ebenso können Gemische der vorgenannten Lösungsmittel verwendet werden, insbesondere technisch verfügbare Destillationsschnitte wie Hexan-technisch, Petrolether 50-70, 60-80, 70-90, 90-110, sowie Kohlendioxid, wenn unter Druck gearbeitet wird. Ebenso können auch unpolare fluorierte Lösungsmittel, z.B. fluorierte C₂-C₈-Alkane bzw. Alkene wie Perfluorethylen, oder fluorierte aromatische Verbindungen wie Hexafluorbenzol und Octafluortoluol verwendet werden. Auch 4-Chlorbenzotrichlorid ist möglich. Bevorzugt wird Hexan (technische Qualität) als Lösungsmittel in Schritt b) eingesetzt.

Die als Lösungsmittel eingesetzten aprotischen Lösungsmittel besitzen üblicherweise einen Wassergehalt von maximal 0,5 Gew.-%, bevorzugt von maximal 0,2 Gew.-%, besonders bevorzugt von maximal 0,1 Gew.-% und insbesondere von maximal 0,05 Gew.-%. Aprotische Kohlenwasserstoffe mit diesem Wassergehalt sind als Lösungsmittel käuflich, können aber auch durch entsprechendes Andestillieren vor der Umsetzung getrocknet werden.

Die Abtrennung der Kupfersalze kann auch unter Druck geschehen, wobei dabei aufgrund des erhöhten Drucks und damit auch des erhöhten Siedepunktes bzw. Siedebereiches des Lösungsmittels bzw. des Lösungsmittelgemisches eine höhere Isolationstemperatur möglich ist und damit gegebenenfalls eine höhere Konzentration an Rohprodukt in dem jeweiligen Lösungsmittel bzw. Lösungsmittelgemisch erzielt werden kann.

Ebenso können zu diesem Zweck neben den inerten organischen Lösungsmittel, die oben genannt sind, unter Druck auch Alkane wie Ethan, Propan, Butan, sowie Kohlendioxid verwendet werden können. Unter erhöhtem Druck kann mit diesen Lösungsmitteln im sogenannten überkritischen Zustand das Produkt von den Kupfersalzen abgetrennt werden, wobei das Produkt in diesem Fall durch Druckverminderung und gleichzeitigem Abdampfen der Lösungsmittel gewonnen wird.

Zur Kristallisation wird die Lösung des Rohprodukts in dem inerten organischen Lösungsmittel von dessen Siedepunkt oder knapp darunter auf einen Temperatur von -40 bis +20°C, bevorzugt - 20 bis +10°C, besonders bevorzugt auf-10 bis +5°C abgekühlt. (Das Abkühlen kann sowohl durch eine Mantelkühlung, als auch durch eine Vakuumsiedekühlung erfolgen.)

Ein wesentliches Merkmal des erfindungsgemäßen Verfahrens ist die besonders hohe Reinheit mit der die Produkte erhalten werden. Nach erfindungsgemäßer Vorgehensweise lassen sich substituierte Benzoylcyanide mit einer Reinheit von >94%, bevorzugt von >95%, besonders bevorzugt von >96% erhalten, wobei gleichzeitig die üblichen Nebenprodukte dieser Reaktion wie die entsprechend substituierte Benzoesäure, sowie das entsprechend substituierte Benzoesäureanhydrid mit einem Gehalt von <2%, bevorzugt von <1%, besonders bevorzugt von <0,6% bzw. mit einem Gehalt von <6%, bevorzugt von < 4%, besonders bevorzugt von <3% erhalten werden.

Die Wirtschaftlichkeit des Verfahren lässt sich weiterhin erhöhen, indem das Lösungsmittel aus der Mutterlauge destilliert wird und erneut verwendet wird.

Ebenso können die abfiltrierten Kupfersalze nach dem an sich bekannten Stand der Technik der Kupferwiedergewinnung zugeführt werden, da diese relativ sauber erhalten werden.

### Beispiele

Alle Reaktionen wurden unter Stickstoffatmosphäre durchgeführt. Die Analyse der erhaltenen Produkte wurde sofern nicht anders angegeben mittels HPLC-Analyse durchgeführt, um zu gewährleisten, dass die üblichen Nebenprodukte wie die entsprechenden Benzoesäuren, sowie deren Anhydride detektiert werden.

**Vergleichsbeispiel 1** (nach GB-A- 2395483)

In einem Kolben wurden 106,9 g (0,5 mol) 2,3-Dichlorbenzoylchlorid bei ca. 35-40°C aufgeschmolzen. Bei dieser Temperatur wurden unter Rühren 62,8 g (0,7 mol) Kupfercyanid zugegeben.

Diese Mischung wurde 6 Stunden lang auf 165°C temperiert, wobei sich die graue Suspension nach braun-grün verfärbte. Das Reaktionsgemisch wurde anschließend auf ca. 80°C abgekühlt. Bei dieser Temperatur wurden 50 ml Toluol zugegeben und die Suspension wurde heiß über eine Glassinternutsche (Pore G3) filtriert. Der Reaktionskolben, sowie der Rückstand in der Nutsche wurde dreimal mit je 20 ml Toluol gespült. Es bleiben 79,0 g feuchter Feststoff auf der Nutsche zurück.

Das aufgefangene Filtrat wurde bei einer Temperatur von 50°C und einem Druck von 25 hPa auf einen Rückstand von 105,3 g eingeengt. Dieser wurde in 650 ml Hexan bei Siedetemperatur (69°C) suspendiert, wobei unlösliche Anteile zurückblieben. Die Suspension wurde bei ca. 60°C klarfiltriert, wobei 21,4 g eines feuchten krümeligen Feststoffs zurückblieben. Eine Analyse des Rückstandes zeigte einen Gehalt von >60% an 2,3-Dichlorbenzoesäureanhydrid. Der Gehalt an 2,3-Dichlorbenzoesäure wurde nicht bestimmt.

Das auf ca. 60°C temperierte Filtrat wurde nun bis auf 15°C abgekühlt, wobei das Produkt in Form gelber Kristalle mit bräunlichen Ölanteilen ausfiel. Dabei beobachtete man, dass ein Teil des Produkt (insbesondere die öligen Anteile) an der Glaswandung des Kolben haftete.

Nach Lösen der Anhaftungen mit einem Spatel wurde die Suspension filtriert und der so erhaltene Feststoff bei einer Temperatur von 40°C und einem Druck von 20 hPa getrocknet. Man erhielt 63,8 g 2,3-Dichlorbenzoylcyanid in Form gelber Kristalle mit Ölanteilen. Die Reinheit des Produkts betrug 89,4 %. Dies entsprach einer Ausbeute von 57 % der Theorie.

Als Nebenkomponente im Produkt konnten noch 6,9 % des entsprechenden 2,3-Dichlorbenzoesäureanhydrids gefunden werden. Der Gehalt an 2,3-Dichlorbenzoesäure wurde nicht bestimmt.

**Vergleichsbeispiel 2** (nach GB-A- 2395483)

In einem Kolben wurden 256,5 g (1,2 mol) 2,3-Dichlorbenzoylchlorid ca. 35-40°C aufgeschmolzen. Bei dieser Temperatur wurden unter Rühren 150,8 g (1,7 mol) Kupfercyanid zugegeben.

Diese Mischung wurde 6 Stunden lang auf 165°C temperiert, wobei sich die graue Suspension nach braun-grün verfärbte. Das Reaktionsgemisch wurde anschließend auf ca. 80°C abgekühlt. Bei dieser Temperatur werden 754 ml Toluol zugegeben und weiter auf 65°C abgekühlt. Bei dieser Temperatur wurde für 3 Stunden ein leichter Stickstoffstrom durch die Lösung geleitet. Anschließend wurde die Suspension heiß über eine Glassinternutsche (Pore G3) filtriert. Der Filtrationsrückstand in der Nutsche wurde zweimal mit je 100 ml Toluol gespült und dann entsorgt.

Das aufgefangene Filtrat wurde bei einer Temperatur von 50°C und einem Druck von 25 hPa auf einen Rückstand von 259,4 g eingeengt. Dieser wurde in 1670 ml Hexan bei Siedetemperatur (69°C) suspendiert, wobei unlösliche Anteile zurückblieben. Die Suspension wurde bei ca. 60°C klarfiltriert, wobei 16,7 g eines feuchten krümeligen Feststoffs zurückblieben. Eine Analyse des Rückstandes zeigte einen Gehalt von >60% an 2,3-Dichlorbenzoesäureanhydrid. Der Gehalt an 2,3-Dichlorbenzoesäure wurde nicht bestimmt.

Das auf ca. 60°C temperierte Filtrat wurde nun bis auf 15°C abgekühlt, wobei das Produkt in Form gelber Kristalle mit bräunlichen Ölanteilen ausfiel. Dabei beobachtet man, dass ein Teil des Produkt (insbesondere die öligen Anteile) an der Glaswandung des Kolben haftete.

Nach Lösen der Anhaftungen mit einem Spatel wurde die Suspension filtriert und der so erhaltene Feststoff bei einer Temperatur von 40°C und einem Druck von 20 hPa getrocknet. Man erhält 185 g 2,3-Dichlorbenzoylcyanid in Form gelber Kristalle mit Ölanteilen. Die Reinheit des Produkts betrug 84 %. Dies entsprach einer Ausbeute von 66 % der Theorie.

Als Nebenkomponente im Produkt konnten noch 9,8 % des entsprechenden 2,3-Dichlorbenzoesäureanhydrids gefunden werden. Der Gehalt an Säure wurde nicht bestimmt.

**Vergleichsbeispiel 3** (in Anlehnung an DE -A-2708182/ DE-A-2624891)

In einem Kolben wurden 53,4 g (0,25 mol) 2,3-Dichlorbenzoylchlorid bei ca. 35-40°C aufgeschmolzen. Bei dieser Temperatur wurden unter Rühren 14,7 g (0,3 mol) Natriumcyanid und 2,2 g (0,03 mol) Kupfercyanid zugegeben.

Diese Mischung wurde 6 Stunden lang auf 165°C temperiert. Die Analyse der Rohmischung zeigte folgendes Ergebnis:

51,8 % 2,3-Dichlorbenzoylchlorid, 38,5 % 2,3-Dichlorbenzoylcyanid und 9,0 % 2,3-Dichlorbenzoesäureanhydrid. Der Gehalt an 2,3-Dichlorbenzoesäure wurde nicht bestimmt.

Das Reaktionsgemisch wurde anschließend weitere 4 Stunden bei 165°C gerührt und auf ca. 80°C abgekühlt. Bei dieser Temperatur wurden 157 ml Toluol zugegeben und die Suspension wurde heiß über eine Glassinternutsche (Pore G3) filtriert. Der Filtrationsrückstand in der Nutsche wurde zweimal mit je 20 ml Toluol gespült und anschließend entsorgt.

Das aufgefangene Filtrat wurde bei einer Temperatur von 50°C und einem Druck von 10 hPa auf einen Rückstand von 54,2 g eingeengt. Eine Analyse des zurückbleibenden dunklen Öls zeigte folgendes Ergebnis:

24,2 % 2,3-Dichlorbenzoylchlorid, 54,6 % 2,3-Dichlorbenzoylcyanid und 5,0 % 2,3-Dichlorbenzoesäureanhydrid. Der Gehalt an 2,3-Dichlorbenzoesäure wurde nicht bestimmt.

### Beispiel 1 (erfindungsgemäß)

In einem Kolben werden 251,4 g (2,8 mol) Kupfercyanid vorgelegt. Nach Erhitzen des Kolbens auf 45°C werden 427,5 g (2,0 mol) aufgeschmolzenes 2,3-Dichlorbenzoylchlorid unter Rühren zugegeben.

Diese Mischung wurde 4 Stunden lang auf 160°C temperiert, wobei sich die graue Suspension nach grau-grün verfärbt. Das Reaktionsgemisch wurde anschließend auf ca. 60°C abgekühlt. Bei dieser Temperatur wurden 2765 ml Hexan (technische Qualität) zugegeben und die Suspension wurde heiß über eine Glassinternutsche (Pore G3) filtriert. Der Reaktionskolben, sowie der Rückstand in der Nutsche wurde zweimal mit je 240 ml Hexan (technische Qualität) gespült. Es blieb ein grauer Feststoff auf der Nutsche zurück: Trockengewicht: 267,9g, der entsorgt wurde.

Das aufgefangene Filtrat wurde in einem zweiten Kolben für 30 min zum Sieden erhitzt (ca. 69°C) und anschließend auf 0°C abgekühlt. Dabei kristallisierte das Produkt in Form gelber Kristalle mit einer sehr geringen Menge an öligen Anteilen aus. Im Gegensatz zu der Beobachtung in Beispiel 1 befand sich nur noch ein vernachlässigbarer Belag am Kolbenrand.

Diese Suspension wurde filtriert und der so erhaltene Feststoff bei einer Temperatur von 30°C und einem Druck von 8 mbar getrocknet. Man erhält 345,8 g 2,3-Dichlorbenzoylcyanid in Form gelber Kristalle. Die Reinheit des Produkts betrug 97,4 %. Dies entsprach einer Ausbeute von 84 % der Theorie.

### Beispiel 2 (erfindungsgemäß)

In einem Kolben wurden 106,9 g (0,5 mol) 2,3-Dichlorbenzoylchlorid bei ca. 35-40°C aufgeschmolzen. Bei dieser Temperatur wurden unter Rühren 49,3 g (0,55 mol) Kupfercyanid zugegeben.

Diese Mischung wurde 4 Stunden lang auf 165°C temperiert, wobei sich die graue Suspension nach hellbraun verfärbt. Das Reaktionsgemisch wurde anschließend auf ca. 60°C abgekühlt. Bei dieser Temperatur wurden 691 ml Hexan zugegeben und die Suspension wurde heiß filtriert. Der Filterrückstand, 81,6 g feuchter Feststoff, wurde nicht mehr nachgewaschen und verworfen.

Das auf ca. 60°C temperierte Filtrat wurde nun bis auf 15°C abgekühlt. Dabei kristallisierte das Produkt in Form gelber Kristalle mit einer sehr geringen Menge an öligen Anteilen aus. Im Gegensatz zu der Beobachtung in Beispiel 1 befand sich nur noch ein vernachlässigbarer Belag am Kolbenrand.

Die Suspension wurde über eine Umkehr Glassinternutsche filtriert und mit Stickstoff eine Stunde lang trocken geblasen. Man erhielt 76,8 g 2,3-Dichlorbenzoylcyanid in Form gelber Kristalle. Die Reinheit des Produkts betrug 97,5 %. Die Kristalle zeigten einen Trocknungsverlust von <0,2%. Dies entsprach einer Ausbeute von 74 % der Theorie.

Als Nebenkomponente im Produkt konnten noch 1,8 % des entsprechenden 2,3-Dichlorbenzoesäureanhydrids und 0,5 % 2,3-Dichlorbenzoesäure (ggf. rausnehmen) gefunden werden.

### Beispiel 3 (erfindungsgemäß)

In einem Kolben wurden 197,0 g (2,2 mol) Kupfercyanid vorgelegt. Nach Erhitzen des Kolbens auf 45°C wurden 427,5 g (2,0 mol) aufgeschmolzenes 2,3-Dichlorbenzoylchlorid unter Rühren zugegeben.

Diese Mischung wurde 4 Stunden lang auf 160°C temperiert, wobei sich die graue Suspension nach grau-grün verfärbte. Das Reaktionsgemisch wurde anschließend auf ca. 60°C abgekühlt. Bei dieser Temperatur wurden 2765 ml Hexan (technische Qualität) zugegeben und die Suspension wurde heiß über eine Glassinternutsche (Pore G3) filtriert. Der Reaktionskolben, sowie der Rückstand in der Nutsche wurde zweimal mit je 240 ml Hexan (technische Qualität) gespült. Es blieb ein grauer Feststoff auf der Nutsche zurück: Trockengewicht: 212,3g, der entsorgt wurde.

Das aufgefangene Filtrat wurde in einem zweiten Kolben für 30 min zum Sieden erhitzt (ca. 69°C) und anschließend auf 0°C abgekühlt. Dabei kristallisierte das Produkt in Form gelber Kristalle mit einer sehr geringen Menge an öligen Anteilen aus. Im Gegensatz zu der Beobachtung in Beispiel 1 befand sich nur noch ein vernachlässigbarer Belag am Kolbenrand.

Diese Suspension wurde filtriert und der so erhaltene Feststoff bei einer Temperatur von 30°C und einem Druck von 8 mbar getrocknet. Man erhielt 344,6 g 2,3-Dichlorbenzoylcyanid in Form gelber Kristalle. Die Reinheit des Produkts betrug 98,0 %. Dies entsprach einer Ausbeute von 84 % der Theorie.

### Beispiel 4 (Analytikvergleich GC vs HPLC)

Ein Analytikvergleich einer Probe von 2,3-Dichlorbenzoylcyanid (hergestellt nach Vergleichsbeispiel 1) hatte folgendes ergeben (es wurden jeweils zwei Bestimmungen durchgeführt):

| Analytikart | Gehalt an 2,3-Dichlorbenzoylchlorid | Gehalt an 2,3-Dichlorbenzoylcyanid | Gehalt an 2,3-Dichlorbenzoesäure | Gehalt an 2,3-Dichlorbenzoesäureanhydrid |
|---|---|---|---|---|
| GC (1. Best.) | 0,00 | 99,34 | 0,00 | 0,61 |
| GC (2. Best.) | 0,00 | 99,32 | 0,00 | 0,62 |
| HPLC (1. Best.) | 0,05 | 92,61 | 1,69 | 5,52 |
| HPLC (2. Best.) | 0,06 | 92,82 | 1,58 | 5,37 |

Diese Tabelle macht deutlich, dass mittels gaschromatographischer Analyse nicht alle Nebenkomponenten der Reaktion bzw. gegebenenfalls ein zu niedriger Nebenkomponentengehalt angezeigt wird. Mittels HPLC-Analyse werden zuverlässige Ergebnisse erhalten.

## Patentansprüche

1. Verfahren zur Herstellung von substituierten Benzoylcyaniden der allgemeinen Formel (I) 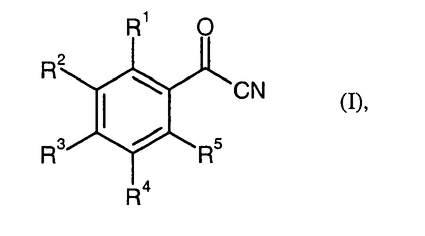 wobei R¹, R², R³, R⁴, R⁵ unabhängig voneinander für Wasserstoff, Chlor, Brom, Jod, Fluor, einen C₁-C₈-Alkyl-, Aryl-, Arylalkyl-, C₁-C₈-Alkoxy-oder C₁-C₈-Alkylmercapto-Rest oder für -CN, -COOR⁶, -CONR₂⁷, -SO₃R⁸, oder -SO ₂NR₂⁹ stehen, wobei R⁶, R⁷, R⁸, R⁹ unabhängig voneinander einem C₁-C₈-Alkylrest entsprechen,
durch
a) Umsetzung von Benzoylchloriden der allgemeinen Formel (II) 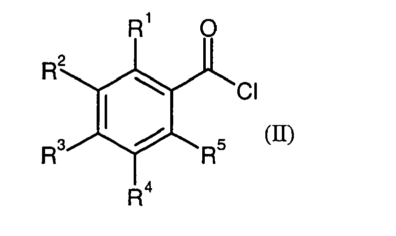 in der
R¹, R², R³, R⁴, R⁵ die gleiche Bedeutung wie in Formel I haben,
mit 0,9-1,4 Moläquivalenten Kupfercyanid ohne weiteres Lösungsmittel, gegebenenfalls unter erhöhten Druck, unter Inertgasatmosphäre bei einer Reaktionstemperatur zwischen 150 und 165°C,
b) wobei nach einer Reaktionszeit von maximal 5 Stunden dem Reaktionsgemisch nach Abkühlung auf eine Temperatur unter 100°C ein aprotisches organisches Lösungsmittel zur Ausfällung des entstandenen Kupfersalzes zugefügt wird und
c) nach Abfiltration des ausfällten Kupfersalzes das verbliebene Filtrat auf eine Temperatur zwischen -40 und +20°C zur Auskristallisation des Rohproduktes abgekühlt wird und
d) das auskristallisierte Rohprodukt vom aprotischen organischen Lösungsmittel abgetrennt und gegebenenfalls getrocknet wird.

2. Verfahren nach Anspruch 1, **dadurch gekennzeichnet, dass** das aprotische organische Lösungsmittel ein C₂-C₈-Alkan, ein gegebenenfalls substituiertes Cycloalkan oder Mischungen daraus, ein fluorhaltiges C₂-C₈ -Alkan bzw. Alken, ein fluorhaltiger Aromat oder Kohlendioxid ist.

3. Verfahren nach Anspruch 1, **dadurch gekennzeichnet, dass** das aprotische organische Lösungsmittel Hexan oder ein technisches Hexan-Gemisch ist.

4. Verfahren nach Anspruch 1, **dadurch gekennzeichnet, dass** das aprotische organische Lösungsmittel ein technischer Destilllationsschnitt wie Petrolether 50-70, Petrolether 60-80, Petrolether 70-90 oder Petrolether 90-110 ist.

5. Verfahren nach einem der vorherigen Ansprüche, **dadurch gekennzeichnet, dass** das substituierte Benzoylcyanid ein halogensubstituiertes Benzoylcyanid ist, bei dem mindestens einer der Reste R1, R2, R3, R4 oder R5 für Chlor, Fluor, Brom oder Jod steht.

6. Verfahren nach Anspruch 5, **dadurch gekennzeichnet, dass** das halogensubstituierte Benzoylcyanid 2,3-Dichlorbenzoylcyanid, 2,4-Dichlorbenzoylcyanid, 2,5-Dichlorbenzoylcyanid, 2,6-Dichlorbenzoylcyanid, 3,4-Dichlorbenzoylcyanid oder 2-Chlorbenzoylcyanid oder ein Isomer davon ist.

7. Verfahren nach einem der vorherigen Ansprüche, **dadurch gekennzeichnet, dass** das Benzoylchlorid der Formel (II) mit 1,0 bis 1,3 Moläquivalenten Kupfercyanid umgesetzt wird.

8. Verfahren nach einem der vorherigen Ansprüche, **dadurch gekennzeichnet, dass** das Benzoylchlorid der Formel (II) mit 1,0 bis 1,1 Moläquivalenten Kupfercyanid umgesetzt wird.

9. Verfahren nach einem der vorherigen Ansprüche, **dadurch gekennzeichnet, dass** die Reaktionstemperatur zwischen 155 und 160°C liegt.

10. Verfahren nach einem der vorherigen Ansprüche, **dadurch gekennzeichnet, dass** die Reaktionszeit maximal 4 Stunden beträgt.

11. Verfahren nach einem der vorherigen Ansprüche, **dadurch gekennzeichnet, dass** die substituierten Benzoylcyanide mit einer Reinheit größer 94 % erhalten werden, die gleichzeitig maximal 2 % der entsprechenden Säurenebenkomponente bzw. maximal 6 % der entsprechenden Anhydridkomponenten enthalten.

12. Verfahren nach einem der vorherigen Ansprüche, **dadurch gekennzeichnet, dass** die substituierten Benzoylcyanide mit einer Reinheit größer 95 % erhalten werden, die gleichzeitig maximal 1 % der entsprechenden Säurenebenkomponente bzw. maximal 4 % der entsprechenden Anhydridkomponenten enthalten.

13. Verfahren nach einem der vorherigen Ansprüche, **dadurch gekennzeichnet, dass** die substituierten Benzoylcyanide mit einer Reinheit größer 96 % erhalten werden, die gleichzeitig maximal 0,6 % der entsprechenden Säurenebenkomponente bzw. maximal 3 % der entsprechenden Anhydridkomponenten enthalten.

14. Substituiertes Benzoylcyanid, erhältlich nach einem der vorherigen Ansprüche 1 bis 13.
